# EUROPEAN PATENT SPECIFICATION

(11) **EP 2 585 109 B1**
(45) Date of publication and mention of the grant of the patent: **21.12.2016**
(21) Application number: 11798804.8
(22) Date of filing: 22.06.2011
(51) Int. Cl.: A61K 31/69, A61K 31/7088, A61K 31/7105, A61K 39/395, A61K 38/16, A61K 38/17, A61K 45/06, A61P 1/16, A61P 35/00, A61P 29/00

(54) **COMPOSITIONS FOR USE IN TREATING DRUG-INDUCED LIVER TOXICITY OR LIVER FAILURE**
ZUSAMMENSETZUNGEN ZUR VERWENDUNG BEI DER BEHANDLUNG VON ARZNEIMITTELINDUZIERTER LEBERTOXIZITÄT ODER LEBERINSUFFIZIENZ
COMPOSITIONS POUR UTILISATION DANS LE TRAITEMENT DE LA TOXICITÉ OU L'INSUFFISANCE HÉPATIQUE INDUITE PAR DES MÉDICAMENTS

(30) Priority: 22.06.2010 US 357137 P
(43) Date of publication of application: 01.05.2013
(73) Proprietor: The General Hospital Corporation, Boston, MA 02114 (US); Massachusetts Institute of Technology, Cambridge, MA 02139 (US)
(72) Inventor: PATEL, Suraj J., Boston Massachusetts 02115 (US); MILWID, John M., Cambridge Massachusetts 02139 (US); YARMUSH, Martin L., Newton Massachusetts 02459 (US); KING, Kevin R., Boston Massachusetts 02115 (US)
(74) Representative: Hoppe, Georg Johannes
(86) International application number: PCT/US2011/041363
(87) International publication number: WO 2011/163308

(56) References cited:
- GUO JINSHENG ET AL: "Protective effects of glycyrrhizin on acetaminophen-induced hepatotoxicity in mice", GASTROENTEROLOGY; DIGESTIVE DISEASE WEEK MEETING/107TH ANNUAL MEETING OF THE AMERICAN-GASTROENTEROLOGICAL-ASSOCIATION, ELSEVIER, PHILADELPHIA, PA; LOS ANGELES, CA, USA, vol. 130, no. 4, Suppl. 2, 1 April 2006 (2006-04-01), page A793, XP008166034, ISSN: 0016-5085, DOI: 10.1016/S0016-5085(06)60009-7 [retrieved on 2006-07-22]
- I. B. NICOUD ET AL: "2-APB protects against liver ischemia-reperfusion injury by reducing cellular and mitochondrial calcium uptake", AMERICAN JOURNAL OF PHYSIOLOGY: GASTROINTESTINAL AND LIVER PHYSIOLOGY, vol. 293, no. 3, 12 July 2007 (2007-07-12) , pages G623-G630, XP055090632, ISSN: 0193-1857, DOI: 10.1152/ajpgi.00521.2006
- A. NAIKI-ITO ET AL: "Gap Junction Dysfunction Reduces Acetaminophen Hepatotoxicity with Impact on Apoptotic Signaling and Connexin 43 Protein Induction in Rat", TOXICOLOGIC PATHOLOGY, vol. 38, no. 2, 22 January 2010 (2010-01-22), pages 280-286, XP055090314, ISSN: 0192-6233, DOI: 10.1177/0192623309357951
- ASAMOTO M ET AL: "CONNEXIN 32 DOMINANT-NEGATIVE MUTANT TRANSGENIC RATS ARE RESISTANT TO HEPATIC DAMAGE BY CHEMICALS", HEPATOLOGY, WILEY, USA, vol. 40, no. 1, 1 July 2004 (2004-07-01), pages 205-210, XP009037537, ISSN: 0270-9139, DOI: 10.1002/HEP.20256
- L. TAO ET AL: "2-Aminoethoxydiphenyl Borate Directly Inhibits Channels Composed of Connexin26 and/or Connexin32", MOLECULAR PHARMACOLOGY, vol. 71, no. 2, 1 February 2007 (2007-02-01), pages 570-579, XP055090948, ISSN: 0026-895X, DOI: 10.1124/mol.106.027508
- E RIVEDAL ET AL.: 'Role of PKC and MAP kinase in EGF- and TPA-induced connexi n43 phosphorylation and inhibition of gap junction intercellular communicati on in rat liver epithelial cells' CARCINOGENESIS vol. 22, no. 9, 2001, pages 1543 - 1550, XP055070243
- NJ KANG ET AL.: 'Inhibition of Gap Junctional Intercellular Communication by the Green Tea Polyphenol(-)-Epigallocatechin Gallate in Norm al Rat Liver Epithelial Cells' J. AGRIC. FOOD CHEM. vol. 56, 2008, pages 10422 - 10427, XP055070246
- M VIVIANA ET AL.: 'Peptide inhibitors of intercellular communication' AM J PHYSIOL LUNG CELL MOL PHYSIOL vol. 279, 2000, pages L619 - L622, XP002529577

## Description

### FIELD OF THE INVENTION

The present embodiments relate to compositions for use in methods for treating drug-induced liver failure by contacting liver tissue with a liver-specific gap junction inhibitor; and/or compositions for use in methods of reducing the liver toxicity of an agent by administering a gap junction inhibitor either simultaneously or sequentially with exposure to the agent.

According to the invention, the gap junction inhibitor is 2-aminoethoxydiphenyl-borate (2APB), as defined in the claims.

### BACKGROUND

At the onset of sterile (aseptic) injury, damaged cells stimulate potent inflammatory responses that amplify the overall injury and contribute to tissue and/or organ dysfunction and disease, but little is known about how this process unfolds. Sterile injury and its inflammatory sequelae can result from poisoning or other exposure to toxins, bums, ischemic injury, reperfusion injury, drug-induced nephrotoxicity, allergic contact dermatitis and autoimmune diseases, for example. An important clinical example of sterile injury is drug-induced liver injury, the most common cause of acute liver failure and a significant public health crisis. Therefore, drug safety is an important public health concern that requires the therapeutic benefits of medications to be continuously weighed against their potential toxicities. The liver represents an important target of such toxicity, as it plays a central role in metabolizing exogenous compounds into reactive intermediates.

More specifically, drug-induced liver injury remains the most frequently cited reason for abandoning compounds early in development, withdrawing them from the market after approval, and restricting their dosing and usage in the clinic. Over 900 clinically available drugs, most notably acetaminophen (APAP), induce significant liver injury and fulminant hepatic failure, a condition which is commonly fatal. Unfortunately, therapies for drug-induced liver injury are limited to discontinuing use of the offending drug, supportive care, and ultimately liver transplantation. Specific medical therapies such as N-Acetylcysteine (NAC) are only effective in the particular case of APAP toxicity. Given these limitations, an urgent need exists to develop a generalizable approach for treating and preventing drug-induced liver injury by improving the safety profile of potentially hepatotoxic drugs.

Naiki-Ito et al. disclose in Toxicologic Pathology, 38(2), 2010, 280-286 that gap junction dysfunction reduces acetaminophen-induced hepatotoxicity, which is dependent on Cx32-associated gap junctional functions in the liver.

Asamoto et al. disclose in Hepatology, 40(1), 2004, 205-210 that Connexin 32 dominant-negative mutant transgenic rats are resistant to hepatic damage by D-galactosamine and carbon tetrachloride.

### SUMMARY

The present invention relates to a composition comprising 2-aminoethoxydiphenyl-borate (2APB) for use in treating drug-induced liver toxicity, as well as to the use of a composition comprising 2APB for the manufacture of a medicament for treating drug-induced liver failure by at least one therapeutic agent.

The present disclosure describes a strategy for inhibiting inflammation in a tissue by administering at least one tissue-specific gap junction inhibitor. Gap junction inhibitors include 2-aminoethyoxydiphenyl-borate (2APB), diphenylborinic anhydride (DPBA), Phenytoin, diphenhydramine (DPDM), DPTTD, 18β-glycyrrhetinic acid (18βGA), glycyrrhizic acid (GA), deglycyrrhizinated licorice (DGL), carbenoxolone, a Cx32 mimetic peptide, an anti-Cx26 small molecule, and anti-Cx32 antibody, a Cx26 mimetic peptide, an anti-Cx32 or anti*-Gjb1* polynucleotide, an anti-Cx26 polynucleotide, an anti-Cx26 antibody, calmodulin inhibitor, or a prodrug, isomer, analog or derivative of any of these.

Aspects of the disclosure are also directed to preventing sterile (aseptic) organ injury. In particular, for example, drug-induced liver injury can be prevented, based on inhibition of liver-specific gap junctions that limit the hepatotoxic side effects of drugs, by coformulating such drugs with this new class of hepatoprotectants, or administering the hepatoprotectants simultaneously or consecutively with the drug. Present aspects provide a novel hepatoprotective strategy that targets gap junction communication to prevent amplification of drug-induced organ injury. Gap junction inhibition is an effective therapy for preventing drug-induced liver injury, and a novel drug development strategy in which efficacious but potentially hepatotoxic drugs are co-formulated with hepatoprotective compounds to improve their safety profile.

Hepatotoxic drugs that can be co-formulated or administered in conjunction with the liver-specific gap junction inhibitor 2APB include Acetaminophen, Alpha-methyldopa (Aldomet), Amiodarone, Amoxicillin, Baclofen, Buproprion, Benoxaprofen, Carbamazapine, Cotrimoxazole, Ciprofloxacin, Chlorzoxazone, Dantrolene, Duloxetine, Diclofenac, Erythromycin, Fluconazole or ketoconazole, Flutamide, Flucloxacillin, Felbamate, Hydralazine, Ibuprofen, muran/azathioprine/6-MP, Isoniazid (INH), Ketoconazole, Levofloxacin, Lamotrigine, labetalol, Leukotriene synthase inhibitors, zileuton (Zyflo), Methotrexate, Mercaptopurine, Nitrofurantoin, Nicotinic acid, Perihexilene maleate, Phenylbutazone, Phenytoin, Pravastatin, fluvastatin, simavastatin, lovastatin, Quinidine, Rifampin, Ranitidine, Sulfa medications (especially Septra or Bactrim), Tacrine, Tetracyclines, Trazodone, Tamoxifen, Telithromycin, Ticlid, Valproic acid, Zileutan, iproniazid, ticrynafen, bromfenac, troglitizone, Ibufenac, Dilevalol, Tasosartan, Fialuridine, Alpidem, Tolrestat, Tolcapone, Amineptine, Trovafloxacin, Thioridazine, Pemoline, Ximelagatran, Lumiracoxib, Sitaxentan, Nefazodone, Ebrotidine, and/or Nimesulide.

One aspect of the invention relates to connexin 32 (Cx32) and/or connexin 26 (Cx26): key hepatic gap junction proteins. In particular, Cx32 is an essential mediator of drug-induced liver injury. Mice deficient in Cx32 are protected against liver damage, acute inflammation, and death in response to hepatotoxic drugs. In a particular aspect provides for a novel hepatoprotectant, 2-aminoethyoxydiphenyl-borate (2APB), that acts as selective small molecule inhibitors of Cx32 and prevent liver damage, completely abrogating mortality when coadministered with known hepatotoxins such as acetaminophen. Other Cx32 inhibiting hepatoprotectants may include derivatives, pro-drugs, isomers and analogs of 2APB, diphenylborinic anhydride (DPBA), Phenytoin, diphenhydramine (DPDM), and/or DPTTD. Additional Cx32 inhibitors include Cx32-specific interfering polynucleotides (anti-Cx32 or anti-*Gjb1* polynucleotides) (e.g., interfering RNAs), and Cx32 mimetic peptides. Another aspect is directed to compositions and methods for inhibiting connexin 26 (Cx26), including anti-Cx26 small molecules, anti-Cx26 mimetic peptides, anti-Cx26 antibodies, and/or anti-Cx26 polynucleotides (e.g., interfering RNAs).

### DESCRIPTION OF THE DRAWINGS

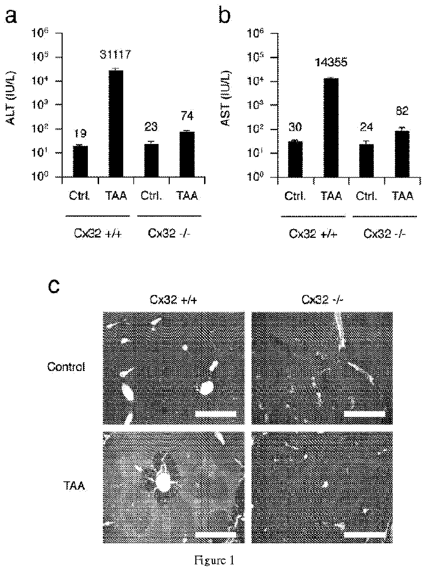

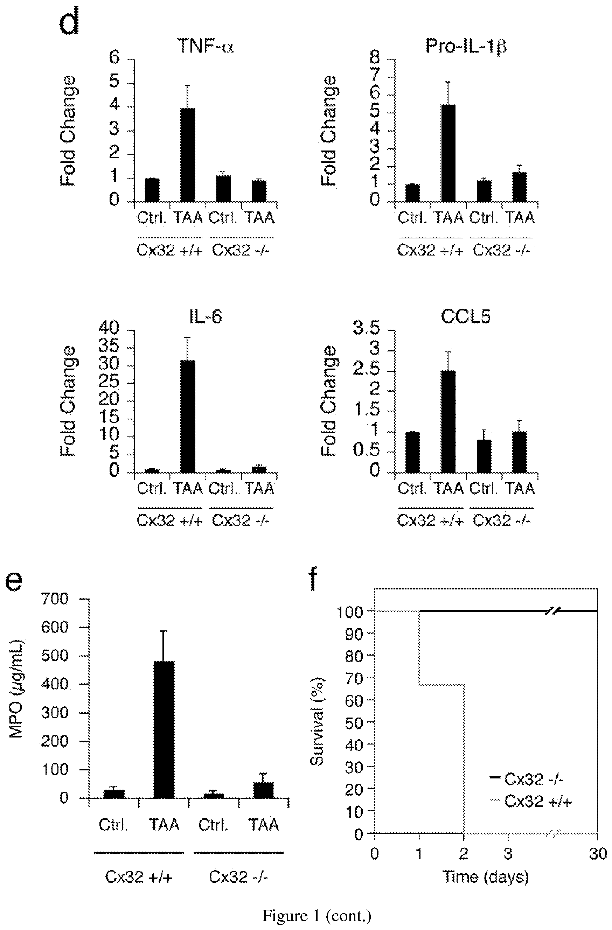

Figure 1 demonstrates that thioacetamide-mediated liver injury is dependent on connexin 32 (Cx32). Figs. 1a and 1b are bar graphs illustrating significantly lower serum transaminase levels in Cx32-/- compared with Cx32+/+ mice 24 hours after treatment with a single sub-lethal dose of the hepatotoxin, thioacetamide (TAA) (200 mg/kg). Fig. 1c is a series of micrographs showing less liver hemorrhaging, necrosis and acute inflammation in Cx32-/- mice compared to Cx32+/+ mice 24 hours after TAA treatment (H&E staining; original magnification 10X; scale bar = 400 µm). Fig. 1d presents bar graphs depicting increase in total liver TNF-α, pro-IL-1α, IL-6, and CCL5 transcripts, as measured by Q-PCR, in Cx32+/+ mice 12 hours after TAA, compared to Cx32-/-mice. Fig. 1e is a bar graph of liver tissue myeloperoxidase activity (MPO) in Cx32+/+ and Cx32-/- mice 24 hours after treatment with TAA. Fig. 1f is a Kaplan-Meier survival curve for Cx32+/+ and Cx32-/- mice over 30 days after a single lethal dose of 500 mg/kg TAA (Cx32+/+ and Cx32-/-: n=12).

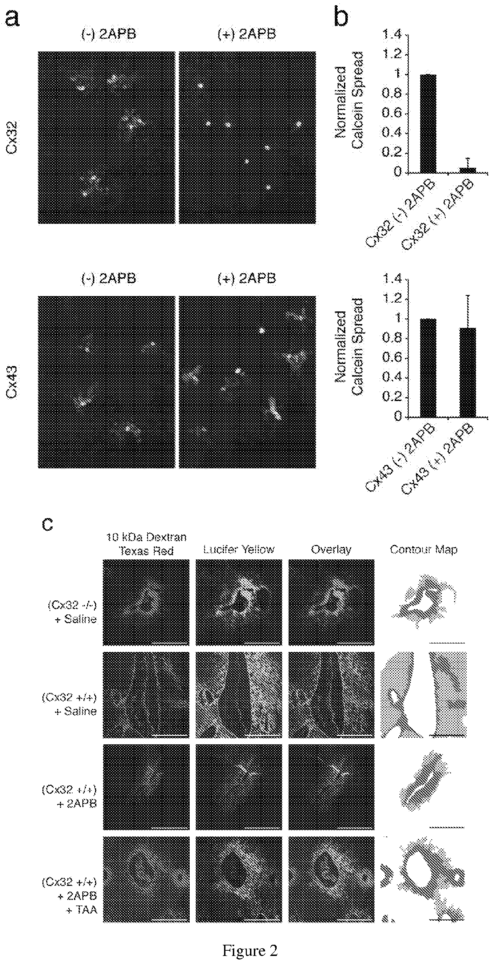

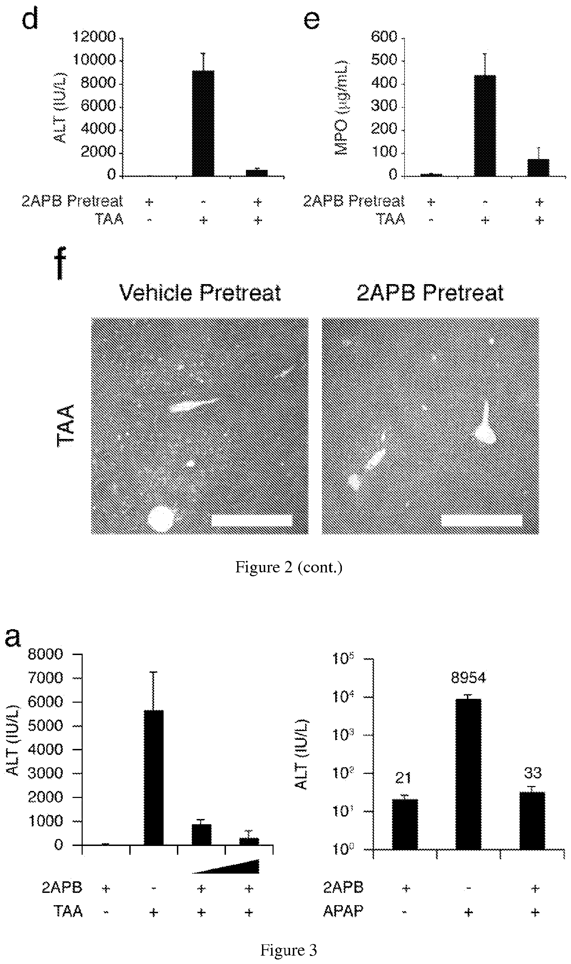

Figure 2 presents data on small molecule inhibitors of Cx32 that selectively block hepatic gap junction communication and limit drug-induced hepatotoxicity. Figs. 2a and 2b depict a dye-coupling parachute assay was used to determine the specificity of 2APB for Cx32 gap junctions compared to Cx43 gap junctions. HeLa Cx32 or Cx43 cells were loaded with gap junction permeable calcein-AM (10 µM) and impermeable CM-DiI (10 µM), and seeded onto unlabeled recipient HeLa Cx32 or Cx43 cells, respectively, in the presence or absence of 2APB (100 µM). After 4 hours, the co-culture was analyzed by fluorescence microscopy (n>5), and gap junction communication was assessed by calculating the average number of calcein positive cells per CM-Dil positive cells, normalized to control conditions without 2APB treatment. Fig. 2c depicts a tissue version of the scrape and load test, developed to evaluate functional gap junction intercellular communication in liver tissue. Cx32+/+ and Cx32-/- mice were treated with saline, 2APB (20 mg/kg), or 2APB plus TAA (200 mg/kg) for 3 hours. Livers were excised, cut into 2-3 mm slices, and a small area of each slice was mechanically damaged with the insertion of a 27 gauge needle coated with 0.5% Lucifer yellow (gap junction permeable) and 0.5% Texas red labeled dextran (gap junction impermeable). Slices were washed, fixed, cryosectioned (7 µm), and analyzed by fluorescence microscopy (n>5) and custom automated image analysis software to produce iso-intensity contour maps outlining spread of Lucifer yellow and dextran-Texas red. Hepatic gap junction connectivity is demonstrated by the spread of gap junction permeable Lucifer yellow compared to gap junction impermeable dextran-Texas red. Fig. 2d: Serum alanine aminotransferase (ALT) levels; Fig. 2e: liver tissue MPO activity; and Fig. 2f: H&E liver sections (original magnification 10X; scale bar = 400 µm) from wildtype mice pretreated with 2APB (20 mg/kg) or vehicle for 60 minutes, and then challenged with TAA (200 mg/kg) for 24 hours.

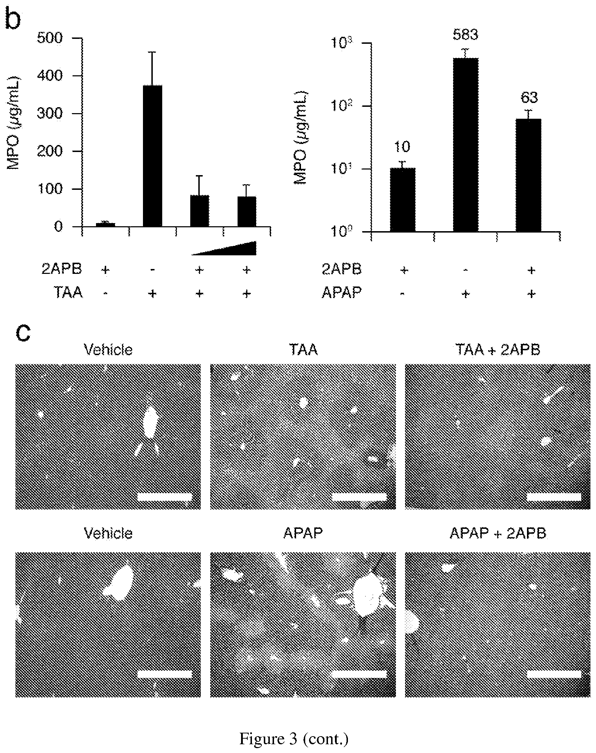

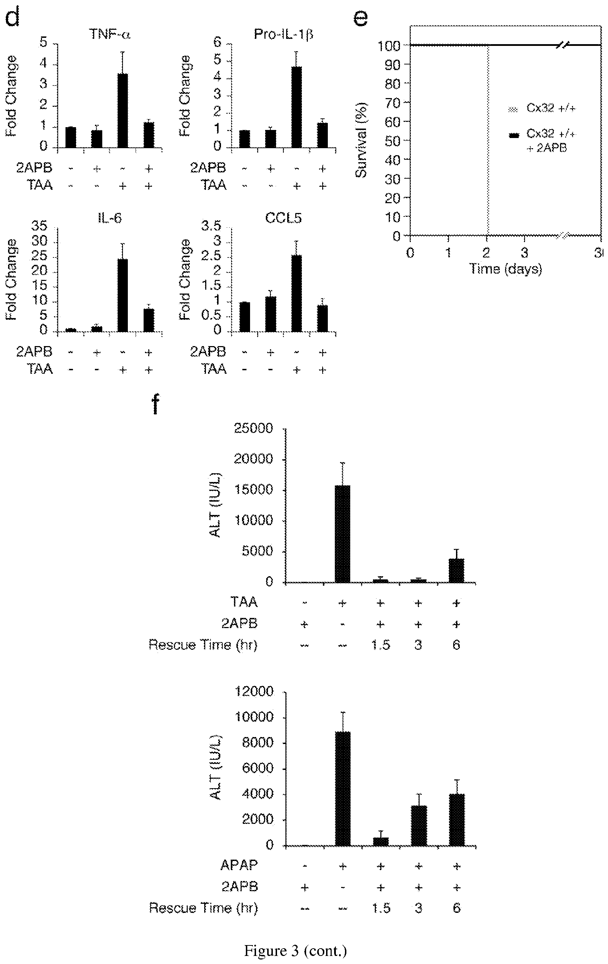

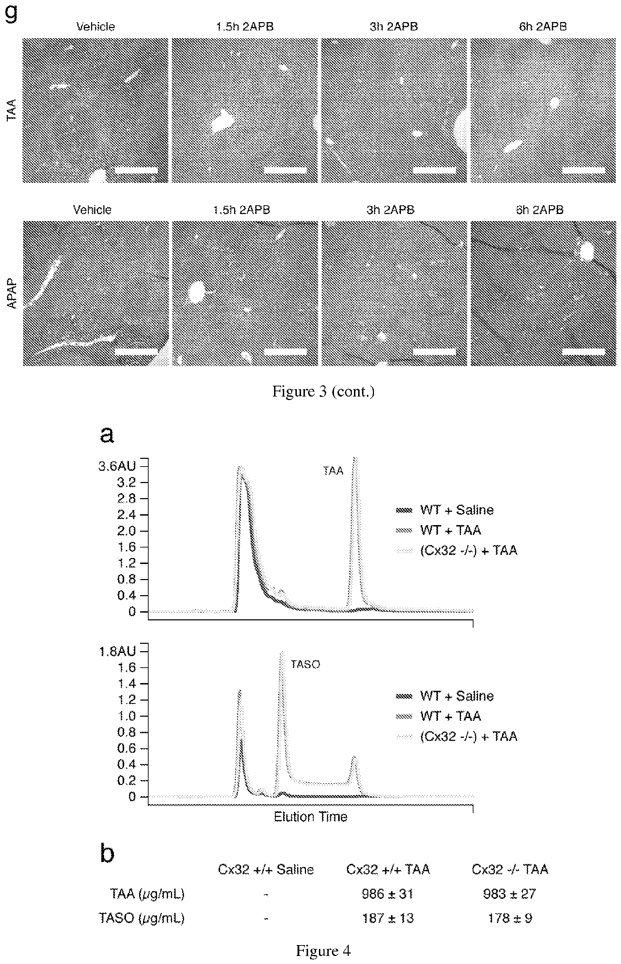

Figure 3 demonstrates that the coadministration of hepatotoxic drugs with Cx32 inhibitors reduces hepatic inflammation, prevents liver failure, and enhances survival. Serum ALT levels (Fig. 3a); liver tissue MPO activity (Fig. 3b); and H&E liver sections (Fig. 3c) (original magnification 10X; scale bar = 400 µm) from wildtype mice after treatment with 2APB alone (20 mg/kg), TAA (200 mg/kg) plus vehicle, TAA (200 mg/kg) plus 2APB (1 or 20 mg/kg), APAP (500 mg/kg) plus vehicle, or APAP (500 mg/kg) plus 2APB (20 mg/kg). Livers and sera were collected 24 hours after TAA challenge, or 16 hours after APAP challenge. Fig. 3d reflects Q-PCR for TNF-α, pro-IL-1β, IL-6, and CCL5 from whole livers of mice treated with TAA and 2APB as described above. Fig. 3e is a Kaplan-Meier survival curve for wildtype mice over 30 days after a single lethal dose of TAA (500 mg/kg) plus vehicle, or TAA (500 mg/kg) plus 2APB (20 mg/kg) (n=10). Serum ALT levels (Fig. 3f) and H&E liver sections (Fig. 3g) (original magnification 10X; scale bar = 400 µm) from wildtype mice after TAA (200 mg/kg) or APAP (500 mg/kg), with or without a rescue injection of 2APB (20 mg/kg) at 1.5, 3 or 6 hours after hepatotoxin challenge. Livers and sera were collected 24 hours after TAA challenge, or 16 hours after APAP challenge.
Figure 4 shows that a deficiency in Cx32 does not affect xenobiotic metabolism. HPLC analysis was performed to demonstrate that the protection against TAA hepatotoxicity in Cx32-/- mice was not a result of defective drug metabolism in the liver. A reverse-phase HPLC assay was used to quantify the serum concentrations of TAA and its toxic metabolite, thioacetamide sulfoxide (TASO), in Cx32+/+ and Cx32-/- treated with saline or TAA (1000 mg/kg) for 90 minutes. Standards were prepared by including known amounts of TAA and TASO in plasma from untreated mice. Equal concentrations of TAA and TASO were found in sera of Cx32+/+ and Cx32-/- mice treated with TAA.

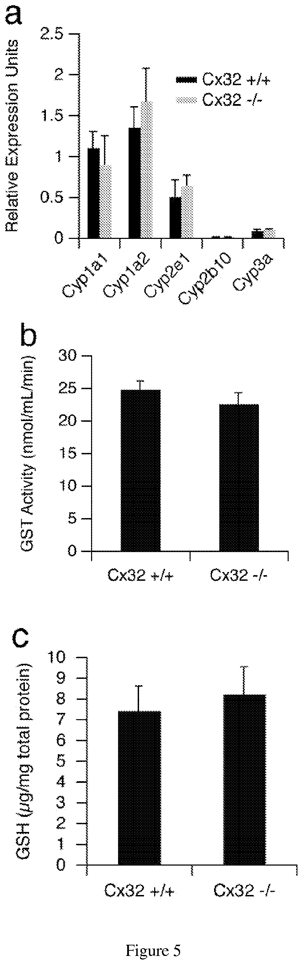

Figure 5 shows that Phase I and II drug metabolism efficiency is similar in Cx32+/+ and Cx32-/- mice. Whole livers were excised from untreated Cx32+/+ and Cx32/ mice and processed for analysis. Fig. 5a presents Q-PCR data for cytochrome P450 enzymes Cyp1a1, Cyp1a2, Cyp2e1, Cyp2b10 and Cyp32, and reveals approximately equal expression in Cx32+/+ and Cx32-/- livers. Fig. 5b: Analysis of glutathione S-transferase (GST) activity shows equal activity in Cx32+/+ and Cx32-/- livers. Fig. 5c: Total glutathione (GSH) content was found to be similar in Cx32+/+ and Cx32-/- livers.

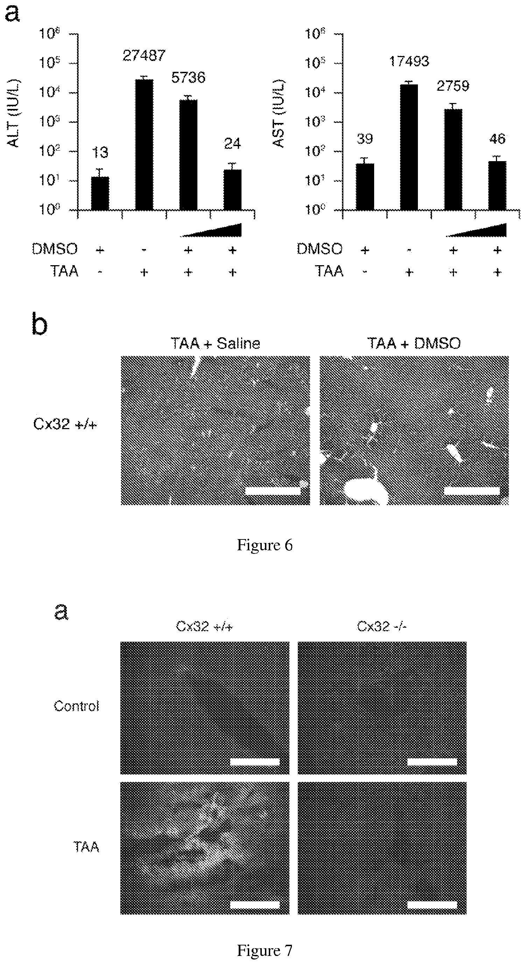

Figure 6 illustrates that drug-induced hepatotoxicity is dependent on oxidative stress. Oxygen free radical scavenger dimethyl sulfoxide (DMSO) (Bruck et al., 35 J. Hepatol. 457 (2001)), was utilized to demonstrate the dependency of TAA-induced hepatotoxicity on oxidative stress. Fig. 6a: Serum transaminase levels in wildtype mice 24 hours after treatment with either saline (1 mL/kg) plus TAA (200 mg/kg) or DMSO (0.1 or 1 mL/kg) plus TAA (200 mg/kg). Fig. 6b: H&E staining of livers (original magnification 10X; scale bar = 400 µm) from wildtype mice 24 hours after treatment with either saline plus TAA, or DMSO plus TAA.

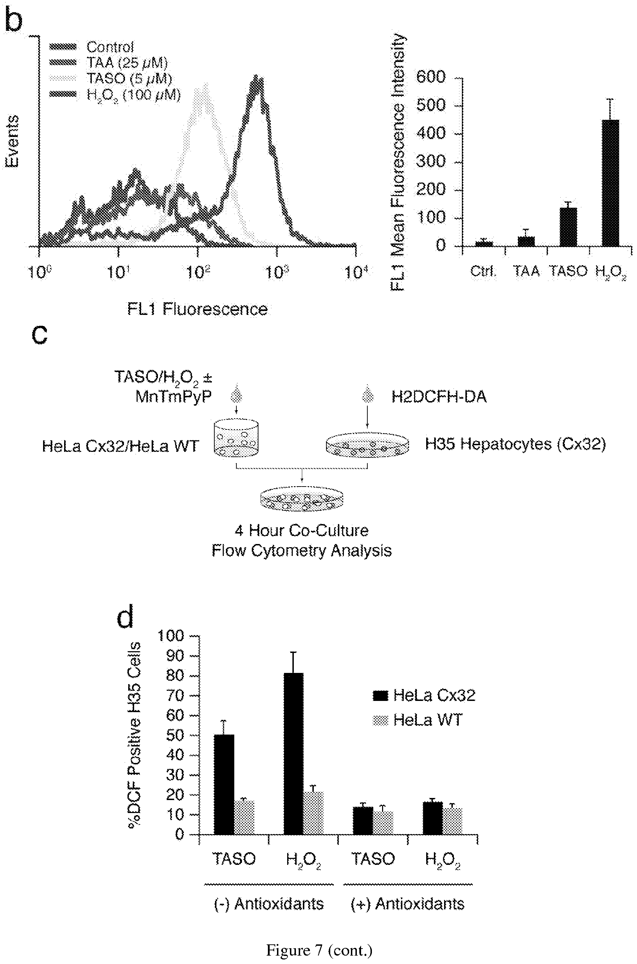

Figure 7 shows that Cx32 gap junctions propagate hepatotoxin-induced free radicals, and amplify the overall burden of liver oxidative stress. Fig. 7a: Freshly prepared liver cryosections (7 µm) from Cx32+/+ and Cx32-/- mice, treated with TAA (200 mg/kg) for 4 hours, were stained with dichlorofluorescein diacetate (H2DCFH-DA), a cell permeable reactive oxygen species (ROS) probe that is converted by intracellular ROS to the fluorescent derivative dichlorodihydrofluorescein (DCF), and analyzed by fluorescence microscopy (original magnification 10X; scale bar = 400 pm; n>5). Fig. 7b: Hepatocyte-derived H35 cells were treated with 25 pM TAA, 5 pM TASO or 100 pM H₂O₂ (positive control) for 2 hours, stained with H2DCFH-DA and analyzed by cytometry for determining levels of intracellular ROS. Flow cytometry distribution of H2DCFH-DA fluorescence and mean fluorescence intensity. Fig. 7c: *In vitro* co-culture system schematic. Wild-type HeLa cells (HeLa WT) deficient in connexin proteins and Cx32-expressing HeLa cells (HeLa Cx32) were stimulated with TASO (5 µM) or H₂O₂ (100 µM; positive control) for 2 hours, in the presence or absence of cell permeable antioxidant/free radical scavenger MnTMPyP (Mn(III)tetrakis(1-Methyl-4-pyridyl)porphyrin pentachloride), and plated onto H35 hepatocytes loaded with ROS probe H2DCFH-DA at a cell ratio of 2:1. Four hours later, the co-culture was analyzed by flow cytometry for gap junction mediated transfer of ROS into H35 cells. Fig. 7d: ROS levels in H35 cells, as determined by flow cytometry.

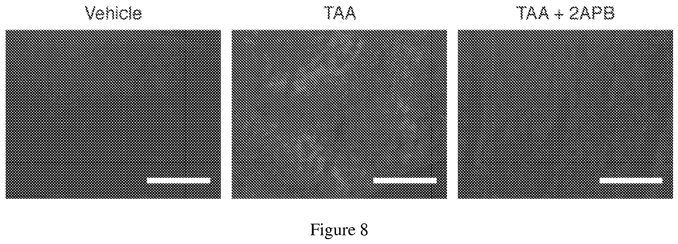

Figure 8 shows that coadministration of TAA with 2-APB abrogated hepatic ROS levels. Liver cryosections from Cx32+/+ mice treated with vehicle, TAA (200 mg/kg) plus vehicle, or TAA (200 mg/kg) plus 2APB (20 mg/kg) for 4 hours were stained with dihydroethidium, a cell permeable free radical probe that binds nucleic DNA and becomes fluorescent when reduced, and analyzed by fluorescence microscopy for ROS activity (n>5).

### DETAILED DESCRIPTION

The terminology used herein is for the purpose of describing particular embodiments only. The scope of the present invention is defined solely by the claims.

Unless defined otherwise, all technical and scientific terms used herein have the same meaning as those commonly understood to one of ordinary skill in the art to which this invention pertains.

Sterile (aseptic) organ injury is a common and dangerous complication that can result from, for example, ingestion or other exposure to toxins, burn injury, ischemic injury, reperfusion injury, drug-induced nephrotoxicity and/or hepatotoxicity, allergic contact dermatitis, and autoimmune diseases. For example, drug-induced sterile liver injury is the most common cause of acute liver failure in the developed world, accounting for up 50% of all clinical cases. Navarro & Senior, 354 N. Engl. J. Med. 731 (2006). Drug-induced liver injury is also the leading cause for termination of drug development and drug withdrawal from the market. Wysocki & Swartz, 165 Arch. Intern. Med. 1363 (2005). At the onset of drug-induced liver failure, toxic drug metabolites lead to hepatocyte death by oxidative stress and necrosis. Gunawan & Kaplowitz, 11 Liver Dis. 459 (2007). This results in secondary activation of the innate immune system, as the initially distressed hepatocytes stimulate a potent sterile, inflammatory response that spreads from the local cellular microenvironment to the uninjured tissue at-large, triggering parenchymal dysfunction and ultimately liver failure. Kaplowitz, 4 Nat. Rev. Crug. Discov 489 (2005); Liu & Kaplowitz, 2 Expert Op. Drug Metab. Toxicol. 493 (2006); Imaeda et al., 119 J. Clin. Invest., 305 (2009); Liu et al., 127 Gastroenterol. 1760 (2004).

Thus, the pathogenesis of drug-induced hepatotoxicity involves multiple phases, during which a majority of injury results from secondary signaling and an overly exuberant host inflammatory response that amplifies the initial hepatotoxin-triggered tissue damage and limits recovery. Kaplowitz, 2005; Liu & Kaplowitz, 2006; Chen et al., 13 Nat. Med. 851 (2007); Imaeda et al., 2009; Jaeschke, 1 Expert Op. Drug MEtb. Toxicol. 389 (2005). Targeting this secondary amplification of tissue damage offers a general strategy for preventing drug-induced liver injury that not limited to specific hepatotoxins, but instead applicable to a broad range of drugs. Recent work by our group and others has demonstrated a link between hepatic gap junctions, molecular channels composed of connexin proteins (Cx) that enable direct intercellular communication between coupled cells (Segretain & Falk, 1662 Biochim. Biophys. Acta 3 (2004)), and the amplification of liver inflammation (Patel et al., 106 PNAS 12867 (2009); Naiki-Ito et al., 38 Toxicol. Pathol. 280 (2010). As described herein, liver-specific gap junction inhibitors can function as a novel class of 'hepatoprotectants', that can be coadministered with hepatotoxic drugs to prevent liver injury, and therefore may effect the way drugs are developed and administered.

In addition to drug-induced tissue injury, tissue injury that may be treated, according to the present disclosure, can result from exposure to toxins including amanita, arsenic, carbon tetrachloride, vinyl chloride, and alfatoxins. Moreover, some liver injury that was previously termed sterile or aseptic has now been shown to be caused by viruses, and may also be treated according to the present disclosure include viral hepatitis (Hepatitis A, B and/or C virus, Yellowfever). Other disease-related liver injury that may be treated by the compositions and methods of the present disclosure include alcoholic liver disease (ALD), non-alcoholic steatohepatitis (NASH), Wilson's disease, obstructive jaundice, autoimmune hepatitis, non-alcoholic fatty liver disease (NAFLD), and ischemic hepatitis.

By way of background, gap junctions are assemblies of intercellular channels composed of integral membrane connexin (Cx) proteins organized into two subsets, alpha connexins (e.g., Cx43) and beta connexins (e.g., Cx32, Cx26). Homologous connexins oligomerize to form a hemichannel (connexon), and a functional channel is formed when a hemichannel from one subset assembles with a hemichannel of the same subset from an adjacent cell. The resulting gap junctions directly connect the cytosol of the coupled cells, enabling ion exchange, current flow, nutrient flow, and secondary messaging. More than twenty members of the connexin family of genes have been identified. The functions of gap junctions are rather well known in organs composed of excitable cells, such as the propagation of action potentials in the heart, but are less documented in many other organs. Cx32 is the predominant gap junction protein in the human liver: more than 90% of hepatic beta connexins are Cx32, and the remaining beta connexins are typically connexin 26 (Cx26). Inhibiting hepatic gap junction communication may focus on Cx32 and/or Cx26. The present embodiments related particularly to Cx32 may be adapted to Cx26 by those skilled in the art, in light of the teachings herein.

The present disclosure demonstrates that drug-induced liver injury is dependent on gap junction communication that amplifies sterile inflammatory signals generated in response to the initial toxic injury. Mice deficient in hepatic gap junction protein connexin 32 (Cx32) were protected against liver damage, inflammation, and death in response to injury induced by classic hepatotoxic drugs. Administration of these drugs typically results in the production of intracellular free radicals that propagated through gap junctions, damaging naïve surrounding cells and expanding the tissue injury front, thereby establishing the sterile inflammatory response. Coadministration of a pharmacologic Cx32 inhibitor, 2-aminoethyoxydiphenyl-borate (2APB), with the hepatotoxic drugs significantly limited hepatocyte damage and sterile inflammation, and completely abrogated mortality, confirming the importance of hepatic gap junction communication in sterile injury. Thus, inhibition of hepatic gap junctions as a viable novel therapeutic strategy for preventing drug hepatotoxicity and potentially other forms of sterile injury.

The dependency of drug-induced liver damage on Cx32, the predominant gap junction protein expressed in the liver, was examined in mice. Cx32-deficient (Cx32-/-) and wild-type (Cx32+/+) mice were treated with a single dose of the established hepatotoxin thioacetamide (TAA), and later examined for evidence of liver injury. After 24 hours, Cx32+/+ mice had significantly elevated serum ALT/AST levels, indicative of severe hepatocellular damage, whereas Cx32-/- mice exhibited near normal levels of ALT/AST and significantly reduced histological evidence of parenchymal damage in the liver (Fig. 1a-1c). The livers of Cx32+/+ mice displayed a classic perivenular pattern of injury that extended into the hepatic lobule, while Cx32-/- mice exhibited only a small localized perivenular ring of injury and necrosis that did not significantly radiate into the hepatic lobule (Fig. 1c). The overall inflammatory response of the liver and the associated recruitment of neutrophils were also attenuated in Cx32-/- mice compared to Cx32+/+ mice (Fig. 1d, 1e). This decreased hepatotoxicity in Cx32-/- mice lead to a dramatic difference in TAA-induced mortality. Treatment of Cx32+/+ mice with TAA resulted in 100% mortality, compared to a 100% survival in Cx32-/- mice (Fig. 1f). That the protective effects of gap junction deficiency were not simply a result of defective drug metabolism in the liver was confirmed by showing that serum concentrations of TAA and its toxic metabolite were the same in Cx32+/+ and Cx32-/- mice, as was phase I cytochrome P450 and phase II GST activity (Figs. 4, 5). Together, these results demonstrate that Cx32 is an essential mediator of drug-induced liver injury, and suggest that Cx32 inhibition might be a viable strategy for preventing hepatotoxic effects of drugs.

Given the unique spatial distribution of both the injury and protection observed in the livers of Cx32+/+ and Cx32-/- mice, respectively, gap junctions might promote the propagation and amplification of an injury signal after TAA treatment. Because oxidative stress is a well-established consequence of hepatotoxin exposure (Jaeshke et al., 65 Toxicol. 166 (2002); Ferret et al. 33 Hepatol. 1173 (2001)), and reactive oxygen species (ROS) are known to propagate through gap junctions (Azzam et al., 22 Oncogene 7050 (2003)), gap junctions might amplify liver injury by facilitating the propagation of ROS and oxidative stress throughout the parenchyma. The dependence of drug-induced liver damage on oxidative stress was demonstrated (Fig. 6), and then compared the burden of intracellular ROS in the livers of Cx32+/+ and Cx32-/- mice. Livers of TAA-treated Cx32+/+ mice exhibited intense focal regions of ROS activity, while those of Cx32-/- mice exhibited comparatively little activity (Fig. 7a). Next, an *in vitro* co-culture system was devised to test whether hepatotoxin-exposed cells require Cx32 to propagate ROS to surrounding unexposed neighbors (Fig. 7b, 7c). Connexin-deficient HeLa cells (HeLa WT) and Cx32-expressing HeLa cells (HeLa Cx32) were stimulated with the reactive metabolite of TAA (Thioacetamide sulfoxide; TASO) in the presence or absence of free radical scavengers (Ferret et al., 2001), and plated onto the Cx32-expressing hepatocyte-derived H35 cells preloaded with a fluorescent ROS probe (Fig. 7c). Using this co-culture system, ROS was detected in unexposed neighbors only when they came into contact with TASO-exposed cells expressing Cx32, and this effect could be abrogated by pretreating the TASO-exposed cells with free radical scavengers (Fig. 7d). Together these results suggest that Cx32 gap junctions propagate hepatotoxin-induced free radicals, and amplify the overall burden of liver oxidative stress.

The pharmaceutical potential of Cx32 inhibition for preventing hepatotoxicity was investigated by screening chemical libraries for small molecule inhibitors of Cx32 that could be administered to wild-type mice to achieve the same hepatoprotection observed in the Cx32 deficient mice. This identified 2-aminoethoxydipenyl-borate (2APB), a compound shown previously to transiently inhibit Cx32 gap junctions *in vitro.* Tao & Harris, 71 Mol. Pharmacol. 57 (2007). The specificity of 2APB for Cx32 gap junctions was determined herein by performing a calcein-AM dye-coupling "parachute" assay using Cx32 and Cx43 expressing HeLa cells (HeLa Cx32, HeLa Cx43). Treatment with 2APB blocked the spread of calcein via Cx32 gap junctions, but had minimal effect on Cx43 gap junctions (Figs. 2a, 2b). The classic "scrape and load" gap junction intercellular communication assay was then adapted to explanted liver slices from Cx32+/+ and Cx32-/- mice, and demonstrated that 2APB effectively blocks hepatic gap junction communication in vivo (Fig. 2c). When wild type mice were pretreated with a single dose of 2APB 60 minutes prior to challenge with TAA, the response was strikingly similar to that observed in Cx32-/- mice. Compared to vehicle-treated mice, those that received 2APB exhibited significantly reduced serum ALT, dramatically reduced histological evidence of parenchymal damage, and limited neutrophil infiltration (Figs. 2d-2f). These findings demonstrate that 2APB is an effective hepatoprotectant, and that targeting the Cx32 gap junction pathway with small molecule inhibitors is a viable strategy for treating and/or preventing drug-induced liver damage.

Unfortunately, pretreatment strategies for reducing drug hepatotoxicity have limited practical utility in both drug development and the clinic. The present invention provides for a coadministration strategy, in which potentially hepatotoxic drugs are co-formulated with the hepatoprotectant 2APB to improve their safety profiles through by reducing drug-induced liver damage. For example, APAP, the active ingredient in several common over-the-counter and prescription pain medications, induces hepatotoxicity that is the most common cause of death due to acute liver failure, and thus represents a significant public health problem. Chun et al., 43 J. Clin. Gastroenterol. 342 (2009). When known hepatotoxic drugs (APAP or TAA) were coadministered with the hepatoprotectant 2APB to wild type mice, and their responses compared with mice receiving APAP or TAA alone, coadministration of 2APB with either TAA or APAP dramatically reduced serum ALT, total hepatic ROS levels, histological evidence of hepatic necrosis and hemorrhaging, liver inflammation, and neutrophil infiltration to near normal levels (Fig. 3; Fig. 9). Remarkably, coadministration of TAA with 2APB resulted in 100% survival, compared to 100% mortality in mice that received TAA with vehicle (Fig. 3e). Together, these results define a novel pharmaceutical and therapeutic strategy in which potential hepatotoxic drugs are co-formulated with hepatoprotectants such as the Cx32-inhibitors, to improve the overall safety profile of drugs and reduce the clinical incidence of drug-induced liver injury.

Although co-formulation of hepatotoxic compounds with hepatoprotectants is an attractive strategy for future drug development, it is not directly applicable to the numerous patients presenting clinically after ingestion hepatotoxic doses of compounds such as APAP. Therefore, whether Cx32 inhibition could rescue from hepatotoxicity after drug ingestion was examined. Single injections of 2APB were given at various times after challenge with either APAP or TAA. Mice treated with 2APB 1.5 hours after either APAP or TAA administration showed a nearly complete absence of hepatotoxicity (Figs. 3d, 3f). Even 6 hours after APAP or TAA challenge, when hepatic necrosis is already evident in control APAP and TAA-treated mice, 2APB treatment reduced serum ALT levels and limited hepatocellular damage and necrosis (Figs. 3f, 3g). These findings demonstrate that hepatic gap junction inhibition, with potent small compounds such as 2APB, successfully rescues from drug-induced liver injury and may provide a clinically useful means to treat liver injury associated with APAP and other hepatotoxic drugs.

Thus, as used herein, treatment or treating refers to limiting tissue damage, ameliorating or lessening symptoms, enhancing the effect of other modalities of treating tissue toxicity, or otherwise affecting or controlling a clinical indication in a positive fashion, such as resolving symptoms of toxicity.

Drug-induced liver injury is a challenging problem facing drug developers, clinicians, and patients. It leads to the abandonment of compounds early in development, withdrawal from market after approval, and restrictive clinical usage. Lee, 349 N. Engl. J. Med. 474 (2003); Kaplowitz, 2005. Approximately 40% of potential drug candidates fail because of unacceptable levels of hepatotoxicity, and many clinically available drugs induce significant liver injury. Wysowski & Swartz, 2005. The present embodiments provide for a new hepatoprotective strategy: Cx32 gap junctions are essential mediators of drug-induced liver injury, as they propagate oxidative stress through the parenchyma, resulting in amplified liver injury. Aspects of the disclosure also provide for targeted inhibition of hepatic gap junctions with small molecule compounds, such as 2-APB, which can protect against, and even rescue from, fatal drug-induced liver injury.

Drugs known to cause varying degrees of hepatotoxicity (often related to dose) include Acetaminophen, Alpha-methyldopa (Aldomet), Amiodarone, Amoxicillin, Baclofen, Buproprion, Benoxaprofen, Carbamazapine, Cotrimoxazole, Ciprofloxacin, Chlorzoxazone, Parfon-forte, Dantrolene, Duloxetine, Diclofenac, voltaren, Erythromycin, Fluconazole or ketoconazole, Flutamide, Flucloxacillin, Felbamate, Hydralazine, Ibuprofen, muran/azathioprine/6-MP, Isoniazid (INH), Ketek, Ketoconazole, Levofloxacin, Lamotrigine, labetalol, Leukotriene synthase inhibitors (asthma medications) [Zafirlukast Accolate] and zileuton (Zyflo), Methotrexate, Mercaptopurine, Nitrofurantoin, Nicotinic acid, Perihexilene maleate, Phenylbutazone, Phenytoin, Pravastatin, fluvastatin, simavastatin, lovastatin, Quinidine, Rifampin, Ranitidine, Sulfa medications (especially Septra or Bactrim), Tacrine, Tetracyclines, Trazodone, Tamoxifen, Telithromycin, Ticlid, Valproic acid, and Zileutan.

During the drug development process, even a small number of drug-induced liver injury events observed in clinical trials raise serious questions about a drug's overall safety profile when expanded to the larger population. Wysowski & Swartz, 2005. Drugs withdrawn from the market or failing in clinical trials because of hepatotoxicity include iproniazid, ticrynafen, benoxaprofen, bromfenac, troglitizone, Ibufenac, Dilevalol, Tasosartan, Fialuridine, Ticrynafen, Alpidem, Tolrestat, Tolcapone, Amineptine, Trovafloxacin, Thioridazine, Pemoline, Ximelagatran, Lumiracoxib, Sitaxentan, Nefazodone, Ebrotidine, and Nimesulide.

To address this problem, an aspect of the disclosure provides for co-formulating potentially hepatotoxic compounds with hepatoprotectants such as gap junction inhibitors to improve drug safety profiles. Drug co-formulation itself is not a novel concept, and has been successfully used in the area of infectious disease, oncology, and cardiovascular medicine, but the strategy has traditionally been used to improve efficacy rather than reduce toxicity. Woodcock et al., 364 N. Engl. J. Med. 985 (2011). Thus, according to an embodiment of the present invention, a drug that has exhibited hepatotoxicity, or been withdrawn or failed in clinical trials because of hepatotoxicity (such as one of those listed herein or another such drug), is combined and or co-formulated with the hepatic-specific gap junction inhibitor 2APB, to reduce hepatotoxicity. This allows for increased safety and/or re-purposed clinical therapeutic utility of such drugs.

Similar Cx32-inhibiting hepatoprotectants that may be used according to the present disclosure include derivatives, isomers, pro-drugs and analogs of 2APB, including, for example, diphenylborinic anhydride (DPBA), Phenytoin, diphenhydramine (DPDM), and DPTTD. *See, e.g.,* Goto et al., 47 Cell Calcium 1 (2010). Generally speaking, a prodrug is a pharmacological substance (drug) administered in an inactive or less active form, that upon administration is metabolized *in vivo* into an active metabolite. Analogs, as envisioned herein, include structural analogs (compounds with a slightly altered chemical structure) and functional analogs (compounds with similar properties). Derivatives refer to compounds that are derived from a compound that is not similar in chemical or physical process. For example, chemical derivatives of 2APB can be formulated in such a way to make them highly lipophilic and more drug-like. The amount of small molecule administered can be determined based on clinical modeling and trials. For example, 2APB may be administered at 20 mg/kg/day, which dose can be adjusted based on clinical observation.

Lending support for the use of Cx32 inhibitors in the treatment of gap junction related tissue injury, it should be noted that there are gap junction inhibitors in clinical development for other indications. These include Tonabersat (SB-220453), a novel benzoylamino-benzopyran, for treating epilepsy and migraines (Proximagen, London, England) (*see* Parsons et al., 132 Br. J. Pharmacol. 1549 (2001)); and Danegaptide (ZP1609, [2S,4R]-1-[2-Aminoacetyl]-4-Benzamidopyrrolidine-2-Carboxylic Acid), a small modified dipeptide inhibitor of cardiac gap junctions for treating life threatening arrhythmias post myocardial ischemia (Zealand Pharma, Copenhagen, Denmark) (*see* Piatnitski Chekler et al., 19 Bioorganic & Med. Chem. Letts. 4551 (2009)). Metastasis inhibitor-22 and MI-18, derivatives of oleamide, specifically inhibit Cx26-mediated formation of gap junction between cells. MI-22 is available from Wako Chemicals USA, Inc. (Wako, TX). Protonated taurin and aminosulfonates can also directly and reversibly inhibit Cx26 channels. Tao & Harris, 279 J. Biol. Chem. 38544 (2004).

Additional gap junction inhibitors include carbenoxolone, 18β-glycyrrhetinic acid (18βGA), glycyrrhizic acid (GA), and deglycyrrhizinated licorice (DGL). The latter three compounds are prepared from saponins isolated from licorice roots, and have been used in herbal or holistic treatments of liver disease and other tissue injury and inflammation for quite some time. *See, e.g.,* Kao et al., 58 J. Agric. Food Chem. 8623 (2010); Santicioli & Maggi, 129 Br. J. Pharmacol. 163 (2000); Chandler, 118 Canadian Pharma. J. 420 (1985).

Additional gap junction inhibitors include Cx32 mimetic peptides. For example, it has been reported that peptides mimicking a sequence on the intracellular loop of Cx32 can block Cx32 function. The connexin mimetic peptides GHGDPLHLEEVKC (SEQ ID NO:1) (intracellular loop, position 110-122) and SRPTEKTVFT (SEQ ID NO:2) (extracellular loop 2, position 182-191) were synthesized by solid-phase chemistry and purified by HPLC. *See* De Vuyst et al., 25 EMBO J. 34 (2006). Cx32 peptides are available commercially, for example, from Novus Biologics (Littleton, CO).

Other gap junction inhibitors include polynucleotides or oligonucleotides that interfere with Cx32 transcription or translation. An "anti-connexin polynucleotide" decreases or inhibits expression of connexin mRNA and/or protein. Anti-connexin polynucleotides include antisense compounds such as antisense polynucleotides, other polynucleotides (such as polynucleotides having siRNA or ribozyme functions). Suitable examples of an anti-connexin polynucleotide include an antisense polynucleotide to a tissue-specific connexin. Accordingly, suitable anti-connexin polynucleotides include, for example, antisense polynucleotides (e.g., Cx32 antisense polynucleotides or Cx26 antisense polynucleotides) that modulate expression or activity of connexins and gap junctions in selected tissues, cells, and subjects. RNA interference can be systemically administered to humans, targeted to tissues, and has been shown to inhibit gene expression in humans *in vivo. See, e.g.,* Davis et al., 464 Nature 1067 (2010).

Anti-connexin polynucleotides include Cx antisense polynucleotides as well as polynucleotides which have functionalities which enable them to downregulate Cx expression (for example, by downregulation of mRNA transcription or translation). In the case of downregulation, this reduces direct inter-cellular communication by gap junctions at the site at which Cx expression is down-regulated. Suitable anti-connexin polynucleotides include RNAi polynucleotides and siRNA polynucleotides. Synthesis of antisense polynucleotides and other anti-connexin polynucleotides such as RNAi, siRNA, and ribozyme polynucleotides as well as polynucleotides having modified and mixed backbones is known to those of skill in the art. *See e.g*., APPLIED ANTISENSE OLIGONUCLEOTIDE TECHNOLOGY (Stein & Krieg, eds, Wiley-Liss, 1998); WO 2010/105209; WO 2010/101951; WO 2010/099341.

More specifically, in humans, the gene encoding connexin 32, *GjB1* is located on the long (q) arm of the X chromosome at position 13.1, and the sequence is known. *See* NCBI Gene ID: 2705. Examples of short interfering RNAs (siRNAs) targeting human Cx32 (target sequence 5'-GCCTAGAATGTTACACATTAA-3') (SEQ ID NO:3), include 5'-CUGAAAUGUUACACAUUAA-dtdt-3' (SEQ ID NO:4) (sense), and 5'-UUAAUGUGUAACAUUUCAG-dGdC-3' (SEQ ID NO:5) (antisense). *See* Hagiwara et al., 153 Br. J. Pharmacol. 1373 (2008); Sato et al., 46 Mol. Carcinog. 215 (2007). Another example oligodeoxynucleotide to connexin 32 is: 5'-TTTCTTTTCTATGTGCTGTTGGTGA (SEQ ID NO:6). An example oligodeoxynucleotide to Cx26 is: 5'-TCCTGAGCAATACCTAACGAACAAATA (SEQ ID NO:7). *See* U.S. Patent Pub. No. 2008/0242631. For a given sequence, a polynucleotide having at least about 70 % homology with that sequence or a polynucleotide which hybridizes to Cx32 mRNA or Cx26 mRNA under conditions of medium to high stringency is also a anti-Cx32 or anti-Cx26 polynucleotide, respectively. GjB1-siRNAs are also available commercially from, for example, OriGene (Rockville, MD), GeneCopoeia (Rockville, MD), and Santa Cruz Biotechnology (CA); as are siRNA expression vectors and vectors for effective delivery of siRNA (e.g., GenScript USA, Inc., Piscataway, NY).

Still other gap junction inhibitors are antibodies, such as anti-Cx32 antibodies or anti-Cx26 antibodies. Antibodies, as used herein means intact immunoglobulin molecules as well as portions, fragments, peptides and derivatives thereof such as, for example, Fab, Fab', F(ab')₂, Fv, CDR regions, paratopes, or any portion or peptide sequence of an antibody that is capable of binding an antigen or epitope, and includes polyclonal antibodies, monoclonal antibodies, chimeric antibodies, fully humanized antibodies, recombinant antibodies, and monoclonal antibodies produced by transgenic animals or portions fragments, peptides and derivatives thereof. An antibody is said to be "capable of binding" a molecule if it is capable of specifically reacting with the molecule to thereby bind the molecule to the antibody. Numerous antibodies have been approved for use in humans. *See, e.g.,* Walden, 9 Nat. Med. 269 (2004). Several antibodies directed to Cx32 are available from commercial sources, including Millipore (Billerica, MA) and LifeSpan BioSciences, Inc. (Seattle, WA). Anti-human Cx26 antibody is also available commercially, for example, from Invitrogen Corp. (Carlsbad, CA).

That gap junction inhibition potently prevents drug-induced liver injury, raises important questions about disease pathogenesis. For example, it is well established that drug-induced liver injury begins as a spatially heterogeneous process that is subsequently propagated to the entire liver. The mechanistic details of how this propagation proceeds were not well understood, however. The present disclosure shows that propagation of drug-induced liver injury is gap junction-dependent, and that Cx32 plays an essential role in amplifying injury, making it an ideal therapeutic target for hepatoprotection. Gap junctions represent a basic pathway for direct communication between neighboring cells. Although in many organs such as the heart, gap junctions have a clearly identified physiological role; their involvement in the liver is not well understood. Rohr, 62 Cardiovasc. Res. 309 (2004). In the context of the heart, transient gap junction inhibition therapies are already currently in clinical trials for preventing life threatening myocardial ischemia reperfusion injury and cardiac arrhythmias. Burashnikov & Antzelevitch, 7 Nat. Rev. Cardiol. 139 (2010); Wit & Duffy, 294 Am. J. Physiol. Heart Circ. Physiol. H16 (2008). Likewise, treating retinal diseases such as diabetic retinopathy and glaucoma with small molecule gap junction inhibitors for minimizing retinal ischemia is presently in preclinical trials in the U.S. Das et al., 373 Biochem. Biophys. Res. Commun. 504 (2008). Understanding the spatiotemporal evolution of drug-induced liver injury from perivenular hepatocytes to neighboring hepatocytes and their local innate immune cells remains an area ripe for investigation. Mechanistically describing injury propagation will likely reveal additional targets for intervention and result in development of novel hepatoprotectants with distinct and complementary mechanisms of action. Together, these families of hepatoprotectants promise to therapeutic toolkit for drug-induced liver injury beyond the toxin-specific therapies such as NAC, to include more generalizable therapies that will prevent fulminant hepatic failure and the need for liver transplantation in response to a broad range of hepatotoxic drugs.

### EXAMPLES

### Example 1. TAA- and APAP-induced hepatotoxicity

C57BL/6 mice were purchased from Jackson Laboratory. Cx32-/- mice were a generous gift from K. Willecke (University of Bonn) and D. Paul (Harvard University). All animal protocols were approved by Massachusetts General Hospital Subcommittee on Research Animal Care. For survival experiments, animals were euthanized when they became moribund according to the criteria of lack of response to stimuli or lack of righting reflex.

TAA (Sigma Aldrich, St. Louis, MO) solution was made fresh for each experiment in 0.9% saline at 20 mg/ml. TAA was dosed at 200, 500 or 1000 mg/kg, depending on the experiment, and injected intraperitoneally. Control mice received the appropriate volume of 0.9% saline. Animals were euthanized by ketamine/xylazine injection at 24 hr for collection of serum and liver tissue for qPCR, GSH/GST assay, MPO activity assay, and histology. For survival experiments, animals were observed every 24 hr for 30 days.

APAP (Sigma Aldrich) solution was made fresh for each experiment in 0.9% saline at 20 mg/ml and heated until dissolved. APAP was dosed at 500 or 750 mg/kg, and injected intraperitoneally after 15 hr of starvation. Animals were euthanized by ketamine/xylazine injection at 16 hr for collection of serum, and liver tissue for MPO activity assay and histology.

### Example 2. 2-Aminoethoxydiphenyl borate treatment

2APB (Sigma Aldrich) was made fresh for each experiment in DMSO as a vehicle at 200 mg/ml. 2APB was dosed at 1 or 20 mg/kg, and was administered before (90 min), with, or after (1.5, 3 or 6 hr) the appropriate dose of TAA or APAP. Vehicle control mice received the appropriate volume of DMSO. Fresh anhydrous DMSO (Sigma Aldrich) was used for each experiment. DMSO was dosed at 0.1 or 1 ml/kg, and coadministered concurrently with 200 mg/kg TAA or saline.

### Example 3. Myeloperoxidase (MPO) activity assay

Mouse liver tissues were homogenized in MPO buffer (0.5% hexadecyl trimethyl ammonium bromide, 10 mM EDTA, 50 mM Na2HPO4, pH 5.4) using a Polytron homogenizer. Liver homogenates were then subject to three freeze-thaw cycles and cleared by centrifugation. MPO reaction was carried out using the Invitrogen EnzChek Myeloperoxidase Activity Assay Kit (Invitrogen, Carlsbad, CA), according to the manufacturer's protocol.

### Example 4. Quantitative RT-PCR

Mouse liver tissues were crushed to a powder in liquid nitrogen, and total RNA was extracted using the Invitrogen Trizol RNA extraction kit, and then purified using the RT2 qPCR-Grade RNA Isolation Kit (SA Biosciences, Frederick, MD), according to the manufacturer's protocol. Total RNA (500 ng) was converted into cDNA using the RT2 First Strand Kit (SA Biosciences). Quantitative RT-PCR was performed using the Stratagene Mx3000P QPCR System and the RT2 qPCR Master Mix Kit (SA Biosciences). Quantitative RT-PCR was performed for mRNA expression of Gapdh, TNFα, pro-IL-1β, IL6, CCL5, Cyp1a1, Cypla2, Cyp2e1, Cyp2b10, and Cyp3a using primers designed by SA Biosciences. Expression of Gapdh was used to standardize the samples, and the results were expressed as a ratio relative to control.

### Example 5. Gap junction communication

For the dye-coupling parachute assay, HeLa Cx32 and HeLa Cx43 cells were grown to confluence, and then double-labeled with 10 µM CM-DiI, a membrane dye that does not spread via gap junctions, and 10 µM calcein-AM, which is converted intracellularly into the gap junction-permeable dye calcein. The labeled cells were then trypsinized, washed, and seeded onto confluent unlabeled recipient HeLa Cx32 or Cx43 cells, respectively, at a 1:200 ratio, in the presence or absence of 2APB (100 µM). The labeled cells were allowed to attach to the monolayer of unlabeled cells and form gap junctions for 4 hr at 37°C and then examined by fluorescence microscopy. For each 2APB experimental condition, the number of unlabeled recipient cells positive for calcein and negative for DiI was determined and normalized to no 2APB control conditions. Connexin 26-, 32-, and 43-expressing HeLa cells were obtained from the University of Bonn.

For tissue scrape and load assay, mice were treated i.p. with saline, 2-APB (20 mg/kg), or 2-APB (20 mg/kg) plus TAA (200 mg/kg), and 3 hr later livers were excised and freshly sliced. A 27-gauge needle was dipped into a solution containing 0.5% Lucifer Yellow (Invitrogen) and 0.5% 10kDa dextran-Texas Red (Invitrogen), and the needle was used to both mechanically damage a small area of each slice and apply the dyes. The liver slices were incubated with the dye solution for 5 minutes, rinsed in saline, fixed in 4% paraformaldehyde for 30 min, frozen in OCT compound, cyro-sectioned into 7 µm sections, rinsed in saline, mounted, and imaged by fluorescence microscopy.

Fluorescence images were captured on a Zeiss 200 Axiovert microscope at a fixed exposure and gain. Images for the tissue scrape load assay were quantified using custom image analysis routines written in MATLAB13. Briefly, images were median filtered, auto-thresholded, and segregated to identify discreet closed regions representing the spread of Lucifer Yellow dye and dextran-Texas Red. Regional outlines were plotted as contour maps by displaying iso-intensity lines at the determined threshold level.

### Example 6. H2DCFH-DA and dihydroethidine hydrochloride (DEH) staining

Freshly cut frozen liver sections (7 µm) were stained with 10 µM H2DCFH-DA (Invitrogen) or 2 µM DEH (Invitrogen) for 30 minutes at 37°C, and imaged by fluorescence microscopy as previously described. King et al., 7 Lab Chip 77 (2007).

### Example 7. Flow cytometry

H35 hepatocyte-derived cells were maintained as described previously. King et al., 2007. Cultured H35 hepatocyte-derived cells were loaded with 10 µM H2DCFH-DA for 30 min at 37°C. This cell-permeable compound is converted into a non-fluorescent product (H2DCF), and oxidized by free radicals to the highly fluorescent dichlorofluoresceine (DCF). Cells were washed in PBS three times, and then treated with saline, TAA (25 µM), TASO (5 µM), or H₂O₂ (100 µM) for 2 hr, or subject to the transplant co-culture assay. After treatment, cells were trypsinized, washed in PBS, and analyzed by flow cytometry.

### Example 8. Transplant co-culture assay

Connexin 32-expressing HeLa (HeLa Cx32) and Cx43-expressing HeLa (HeLa Cx43) cells were stimulated with saline, TASO (5 µM), or H₂O₂ (100 µM) in suspension for 2 hr, in the presence or absence of cell permeable anti-oxidant MnTMPyP (Calbiochem). Two hr after treatment, HeLa cells were washed three times in PBS, counted, and plated onto a sub-confluent layer of H35 cells, preloaded with H2DCFH-DA, at a cell ratio of 2:1. After 4 hr of co-culture, cells were trypsinized and H35 cells were analyzed for ROS activity, as indicated by H2DCFH-DA fluorescence, by flow cytometry.

### Example 9. HPLC-based quantification of TAA and TASO

To quantify TAA and TASO in plasma of mice, a reverse-phase HPLC assay was used, as described previously. Chilakapati et al., 230 Toxicol. 105 (2007). Briefly, 7% acetonitrile, 50 mM sodium sulfate, and 50 mM potassium phosphate buffer was used as the mobile phase. An SPS-ODS column (5 µm; Regis Technologies) was used to separate the components at 1 ml/min. TAA was detected by UV absorption at 212 nm, and TASO at 290 nm, using a photodiode array detector. Retention times for TAA and TASO were approximately 4.1 min and 3 min, respectively. Standards were prepared by including known amounts of TAA and TASO in plasma from untreated mice.

### Example 10. Synthesis of TASO

Thioacetamide S-Oxide (TASO) was synthesized as described previously. Porter & Neal, 6 Drug Metab. Dispos. 379 (1978). Briefly, thioacetamide (TAA) was dissolved in acetone and chilled to -5°C. Then, 30% H₂O₂ was added rapidly, the mixture was agitated thoroughly, and stored at 4°C for 24 hr until the product crystallized. The product, TASO, was recovered by filtration and washed with five portions of cold acetone. The purity was examined by HPLC, as described. *Id.*

### Example 11. Analysis of GST activity and total GSH content

Mouse liver tissues were lysed in 100 mM potassium phosphate, containing 2 mM EDTA, and total protein content was determined. Enzymatic activity toward 1-chloro-2,4-dinitrobenzene (CNDB) (Sigma Aldrich) was assayed in a buffer containing 100 mM potassium phosphate, 0.1% Triton X-100, 1 mM glutathione and 1mM CNDB. Formation of glutathione/CNDB conjugate was measured in a spectrophotometer at 340 nm, as an indicator of GST activity. Total GSH content was measured using the Glutathione Assay Kit (Sigma Aldrich), per the manufacturer's protocol. Briefly, mouse liver tissues were lysed and total protein content was determined. Samples were deproteinized with 5% 5-sulfosalicylic acid, and glutathione content of the samples was assayed using a kinetic assay in which catalytic amounts of glutathione cause a continuous reduction of 5,5'-dithiobis-(2-nitrobenzoic) acid (DTNB) to TNB. TNB was measured colorimetrically at 412 nm, as an indicator of total GSH content.

## Claims

1. A composition comprising 2-aminoethoxydiphenyl-borate (2APB) for use in treating drug-induced liver toxicity.

2. The composition for use according to claim 1, wherein the drug-induced liver toxicity comprises toxicity from a therapeutic agent selected from the group consisting of:
Acetaminophen, Alpha-methyldopa, Amiodarone, Amoxicillin, Baclofen, Buproprion, Benoxaprofen, Carbamazapine, Cotrimoxazole, Ciprofloxacin, Chlorzoxazone, Dantrolene, Duloxetine, Diclofenac, Erythromycin, Fluconazole or ketoconazole, Flutamide, Flucloxacillin, Felbamate, Hydralazine, Ibuprofen, muran/azathioprine/6-MP, Isoniazid (INH), Ketoconazole, Levofloxacin, Lamotrigine, labetalol, Leukotriene synthase inhibitors, zileuton, Methotrexate, Mercaptopurine, Nitrofurantoin, Nicotinic acid, Perihexilene maleate, Phenylbutazone, Phenytoin, Pravastatin, fluvastatin, simavastatin, lovastatin, Quinidine, Rifampin, Ranitidine, Sulfa medications, Tacrine, Tetracyclines, Trazodone, Tamoxifen, Telithromycin, Ticlid, Valproic acid, Zileutan, iproniazid, ticrynafen, bromfenac, troglitizone, Ibufenac, Dilevalol, Tasosartan, Fialuridine, Alpidem, Tolrestat, Tolcapone, Amineptine, Trovafloxacin, Thioridazine, Pemoline, Ximelagatran, Lumiracoxib, Sitaxentan, Nefazodone, Ebrotidine, and Nimesulide.

3. Use of a composition comprising 2-aminoethoxydiphenyl-borate (2APB) for the manufacture of a medicament for treating drug-induced liver failure by at least one therapeutic agent.

4. The use of claim 3, wherein the therapeutic agent is selected from the group consisting of: Acetaminophen, Alpha-methyldopa, Amiodarone, Amoxicillin, Baclofen, Buproprion, Benoxaprofen, Carbamazapine, Cotrimoxazole, Ciprofloxacin, Chlorzoxazone, Dantrolene, Duloxetine, Diclofenac, Erythromycin, Fluconazole or ketoconazole, Flutamide, Flucloxacillin, Felbamate, Hydralazine, Ibuprofen, muran/azathioprine/6-MP, Isoniazid (INH), Ketoconazole, Levofloxacin, Lamotrigine, labetalol, Leukotriene synthase inhibitors, zileuton, Methotrexate, Mercaptopurine, Nitrofurantoin, Nicotinic acid, Perihexilene maleate, Phenylbutazone, Phenytoin, Pravastatin, fluvastatin, simavastatin, lovastatin, Quinidine, Rifampin, Ranitidine, Sulfa medications, Tacrine, Tetracyclines, Trazodone, Tamoxifen, Telithromycin, Ticlid, Valproic acid, Zileutan, iproniazid, ticrynafen, bromfenac, troglitizone, Ibufenac, Dilevalol, Tasosartan, Fialuridine, Alpidem, Tolrestat, Tolcapone, Amineptine, Trovafloxacin, Thioridazine, Pemoline, Ximelagatran, Lumiracoxib, Sitaxentan, Nefazodone, Ebrotidine, and Nimesulide.

## Patentansprüche

1. Zusammensetzung, umfassend 2-Aminoethoxydiphenylborat (2APB), zur Verwendung bei der Behandlung von arzneimittelinduzierter Lebertoxizität.

2. Zusammensetzung zur Verwendung nach Anspruch 1, wobei die arzneimittelinduzierte Lebertoxizität Toxizität von einem Therapeutikum umfasst, dass ausgewählt ist aus der Gruppe bestehend aus: Acetaminophen, alpha-Methyldopa, Amiodaron, Amoxicillin, Baclofen, Buproprion, Benoxaprofen, Carbamazapin, Cotrimoxazol, Ciprofloxacin, Chlorzoxazon, Dantrolen, Duloxetin, Diclofenac, Erythromycin, Fluconazol oder Ketoconazol, Flutamid, Flucloxacillin, Felbamat, Hydralazin, Ibuprofen, Muran/Azathioprin/6-MP, Isoniazid (INH), Ketoconazol, Levofloxacin, Lamotrigin, Labetalol, Leukotriensynthase-Inhibitoren, Zileuton, Methotrexat, Mercaptopurin, Nitrofurantoin, Nicotinsäure, Perihexilenmaleat, Phenylbutazon, Phenytoin, Pravastatin, Fluvastatin, Simavastatin, Lovastatin, Chinidin, Rifampin, Ranitidin, Sulfa-Medikamenten, Tacrin, Tetracyclinen, Trazodon, Tamoxifen, Telithromycin, Ticlid, Valproinsäure, Zileutan, Iproniazid, Ticrynafen, Bromfenac, Troglitizon, Ibufenac, Dilevalol, Tasosartan, Fialuridin, Alpidem, Tolrestat, Tolcapon, Amineptin, Trovafloxacin, Thioridazin, Pemolin, Ximelagatran, Lumiracoxib, Sitaxentan, Nefazodon, Ebrotidin und Nimesulid.

3. Verwendung einer 2-Aminoethoxydiphenylborat (2APB) umfassenden Zusammensetzung zur Herstellung eines Medikaments zur Behandlung von durch mindestens ein Therapeutikum verursachter arzneimittelinduzierter Leberinsuffizienz.

4. Verwendung nach Anspruch 3, wobei das Therapeutikum aus der aus den folgenden bestehenden Gruppe ausgewählt ist: Acetaminophen, alpha-Methyldopa, Amiodaron, Amoxicillin, Baclofen, Buproprion, Benoxaprofen, Carbamazapin, Cotrimoxazol, Ciprofloxacin, Chlorzoxazon, Dantrolen, Duloxetin, Diclofenac, Erythromycin, Fluconazol oder Ketoconazol, Flutamid, Flucloxacillin, Felbamat, Hydralazin, Ibuprofen, Muran/Azathioprin/6-MP, Isoniazid (INH), Ketoconazol, Levofloxacin, Lamotrigin, Labetalol, Leukotriensynthase-Inhibitoren, Zileuton, Methotrexat, Mercaptopurin, Nitrofurantoin, Nicotinsäure, Perihexilenmaleat, Phenylbutazon, Phenytoin, Pravastatin, Fluvastatin, Simavastatin, Lovastatin, Chinidin, Rifampin, Ranitidin, Sulfa-Medikamenten, Tacrin, Tetracyclinen, Trazodon, Tamoxifen, Telithromycin, Ticlid, Valproinsäure, Zileutan, Iproniazid, Ticrynafen, Bromfenac, Troglitizon, Ibufenac, Dilevalol, Tasosartan, Fialuridin, Alpidem, Tolrestat, Tolcapon, Amineptin, Trovafloxacin, Thioridazin, Pemolin, Ximelagatran, Lumiracoxib, Sitaxentan, Nefazodon, Ebrotidin und Nimesulid.

## Revendications

1. Composition comprenant du borate de 2-aminoéthoxydiphényle (2APB) pour utilisation dans le traitement d'une toxicité hépatique d'origine médicamenteuse.

2. Composition pour utilisation selon la revendication 1, la toxicité hépatique d'origine médicamenteuse comprenant une toxicité d'un agent thérapeutique choisi dans le groupe constitué de : l'acétaminophène, l'alpha-méthyldopa, l'amiodarone, l'amoxicilline, le baclofène, le buproprion, le benoxaprofène, la carbamazapine, le cotrimoxazole, la ciprofloxacine, la chlorzoxazone, le dantrolène, la duloxétine, le diclofénac, l'érythromycine, le fluconazole ou le kétoconazole, le flutamide, la flucloxacilline, le felbamate, l'hydralazine, l'ibuprofène, le murane/azathioprine/6-MP, l'isoniazide (INH), le kétoconazole, la levofloxacine, la lamotrigine, le labétalol, les inhibiteurs de leucotriène synthase, le zileuton, le méthotrexate, la mercaptopurine, la nitrofurantoïne, l'acide nicotinique, le maléate de périhéxilène, la phénylbutazone, la phénytoïne, la pravastatine, la fluvastatine, la simavastatine, la lovastatine, la quinidine, la rifampine, la ranitidine, les sulfamides, la tacrine, les tétracyclines, la trazodone, le tamoxifène, la télithromycine, le Ticlid, l'acide valproïque, le zileutan, l'iproniazide, le ticrynafène, le bromofénac, la troglitizone, l'ibufénac, le dilévalol, le tasosartan, la fialuridine, l'alpidem, le tolrestat, la tolcapone, l'amineptine, la trovafloxacine, la thioridazine, la pémoline, le ximélagatran, le lumiracoxib, le sitaxentan, la néfazodone, l'ébrotidine et le nimésulide.

3. Utilisation d'une composition comprenant du borate de 2-aminoéthoxydiphényle (2APB) pour la fabrication d'un médicament pour traiter une insuffisance hépatique d'origine médicamenteuse par au moins un agent thérapeutique.

4. Utilisation de la revendication 3, dans laquelle l'agent thérapeutique est choisi dans le groupe constitué de : l'acétaminophène, l'alpha-méthyldopa, l'amiodarone, l'amoxicilline, le baclofène, le buproprion, le benoxaprofène, la carbamazapine, le cotrimoxazole, la ciprofloxacine, la chlorzoxazone, le dantrolène, la duloxétine, le diclofénac, l'érythromycine, le fluconazole ou le kétoconazole, le flutamide, la flucloxacilline, le felbamate, l'hydralazine, l'ibuprofène, le murane/azathioprine/6-MP, l'isoniazide (INH), le kétoconazole, la levofloxacine, la lamotrigine, le labétalol, les inhibiteurs de leucotriène synthase, le zileuton, le méthotrexate, la mercaptopurine, la nitrofurantoïne, l'acide nicotinique, le maléate de périhéxilène, la phénylbutazone, la phénytoïne, la pravastatine, la fluvastatine, la simavastatine, la lovastatine, la quinidine, la rifampine, la ranitidine, les sulfamides, la tacrine, les tétracyclines, la trazodone, le tamoxifène, la télithromycine, le Ticlid, l'acide valproïque, le zileutan, l'iproniazide, le ticrynafène, le bromofénac, la troglitizone, l'ibufénac, le dilévalol, le tasosartan, la fialuridine, l'alpidem, le tolrestat, la tolcapone, l'amineptine, la trovafloxacine, la thioridazine, la pémoline, le ximélagatran, le lumiracoxib, le sitaxentan, la néfazodone, l'ébrotidine et le nimésulide.
